# EUROPEAN PATENT SPECIFICATION

(11) **EP 4 282 464 B1**
(45) Date of publication and mention of the grant of the patent: **25.09.2024**
(21) Application number: 22175856.8
(22) Date of filing: 27.05.2022
(51) Int. Cl.: A61N 1/36, A61N 1/04, A61B 5/00

(54) **ELECTRODE HELMET FOR ELECTRICAL RECORDING AND/OR STIMULATION**
ELEKTRODENHELM FÜR ELEKTRISCHE AUFZEICHNUNG UND/ODER STIMULATION
CASQUE D'ÉLECTRODE D'ENREGISTREMENT ET/OU DE STIMULATION ÉLECTRIQUE

(43) Date of publication of application: 29.11.2023
(73) Proprietor: Bottneuro AG, 4056 Basel (CH)
(72) Inventor: OSMANI, Bekim, 4056 Basel (CH); JOODAKI, Mahyar, 4054 Basel (CH); KLAAS, Julius, 8048 Zürich (CH)
(74) Representative: Maucher Jenkins Patent- und Rechtsanwälte

(56) References cited:
- US-A1- 2012 265 261
- US-A1- 2017 165 485
- US-A1- 2017 312 517
- US-A1- 2021 015 427
- US-A1- 2021 196 943

## Description

The present disclosure relates to the field of electrical stimulation of brain tissue and/or recording of nerve signals from the brain, in particular for treatment and/or diagnosis of neuronal diseases, for example neurodegenerative diseases such as Alzheimer's disease (AD) but also for stroke rehabilitation, treatment of brain tumors and epilepsy. The invention concerns a helmet to be worn by a patient that can be used for that purpose. The invention also deals with improved methods for designing and fabricating such helmets as a series based on a common design. Finally, the invention also concerns a specific method for fabricating such helmets rapidly and efficiently.

It is well-known that electrical signals produced by nerve cell activity in the human brain can be measured by electroencephalography (EEG) on the surface of the scalp. For this purpose, typically a large number (>10) of recording electrodes, each equipped with an individual cable, are attached to a patient's scalp and an EEG signal is recorded. While this approach can provide accurate EEG signals, it is quite cumbersome to implement (due to the large number of cables that have to be used to electrically connect each of the electrodes) and seldomly used outside of hospitals.

Patient-specific electrical stimulation of brain tissue has also been used intensively in clinical therapy. For this purpose, electrical stimulation devices, capable of delivering electrical fields that penetrate deep into the brain at the right location, are employed. This often requires configuring the stimulation device to the needs of the individual patient (e.g., using finite element methods based on prior patient's MRI data), because the currents injected into the brain should be steered to the right location inside the brain. There have also been endeavours to adapt protocols for electrical stimulation to the needs of the patient.

One method currently used for electrical stimulation and/or recording is to provide a soft bonnet, equipped with a number of individually addressable non-invasive electrodes, which the patient needs to wear during the treatment/the recording, very often for hours (e.g., for up to 18h when monitoring brain waves). One major drawback of this approach is that the position of each electrode has to be checked prior to providing electrical drive voltages to each electrode. Moreover, as the bonnet is soft and not stable in shape, the electrode position relative to the brain may change during wearing of the bonnet, thus posing the risk of stimulating at a wrong location within the brain.

US 2021 015427 A1 describes a helmet featuring an inner shell and an outer shell, wherein the inner shell offers a patient-specific design and the outer shell has a common design, such that the outer shell can be used with different patients. The helmet features multiple magnetic sensors for performing a magnetoencephalography (MEG). These sensors are mounted to the outer shell using mechanical clips, by which each sensor is accurately positioned at a defined static position with respect to the outer shell and thereby also with respect to the skull of the patient wearing the helmet. When the outer shell is used on different patients, each time exchanging the inner shell of the helmet which fits to the individual skull of the respective patient, the individual positions of the sensors with respect to the outer shell remain the same. Starting out from this background, an object of the present invention is to provide a method and appropriate means for enabling more accurate patient-specific electrical stimulation of the brain and/or recording of nerve signals from the brain. In particular, the invention aims at enabling a higher precision of electrical stimulation that is tailor-made to the needs of an individual patient, providing higher spatial resolution in electrical recording and also improving the comfort for the patient during the treatment.

In accordance with the present invention, a helmet is provided (which may be referred to as an "electrode helmet") which can be comfortably worn by a patient on her/his head, in particular due to its patient-specific geometry. In particular, the helmet can show an inner contour surface to be brought into contact with the scalp of the patient wearing the helmet and this inner contour surface can show a patient-specific geometry that matches the geometry of the skull of an individual patient, in particular with respect to a maximum length and/or maximum height and/or maximum width of the skull. As one advantage of this approach, the helmet, unlike other previously known helmets used for similar purposes, does not need to have a chin strap. Rather, the helmet can be held by itself in a predetermined position relative to the patient's head, due to its patient-specific design, in particular said inner contour surface. This means that the helmet no longer has to be aligned with the patient's head, as has been the case with previously known devices.

The helmet comprises a shell (which may form a main body of the helmet and may provide structural stability) designed for carrying a number of m electrodes. Each of these electrodes may be designed for electrically contacting the scalp of a patient, when the patient is wearing the helmet. The electrodes can thus be used for electrical stimulation (i.e., injecting electrical currents into the brain of the patient) or electrical recording (i.e., recording electrical signals from the brain of the patient).

The shell of the helmet is stable in shape, as it can be formed from flexible materials that provide sufficient structural strength, such as polyamide or other suitable polymers. Moreover, the shell offers a patient-specific design. In other words, the shell may have been designed and fabricated according to data which specify the anatomy of a skull of an individual patient for whom the helmet is intended. In particular, the helmet may be designed such that the head of the patient is only in contact with the electrodes, when the patient is wearing the helmet specifically designed for her/him.

For solving the afore mentioned problem, the invention further proposes that said helmet features a number of N electrode holders, with each holder being designed for holding a respective electrode in place. Furthermore, each holder features a mechanical spring for providing a contact force, which is intended for pressing the electrodes into the scalp of the patient, when the patient is wearing the helmet.

This approach not only has the advantage that the wearing comfort can be improved because an ideal fit to the patient's skull can be achieved. Due to the patient-specific geometry of the helmet, it is also possible to define the position of each electrode with respect to a sagittal and a frontal plane running through the head of a patient, as soon as the patient is wearing the helmet (and thereby to the brain of the patient), with high accuracy. As the helmet can only be worn in a specific orientation by the patient, the location of the electrodes also cannot change significantly during the treatment, which is highly beneficial for the safety of the treatment.

As will be explained in greater detail below, the helmet may be designed in such a way that it can only be worn by the patient in one particular orientation with respect to both the sagittal and frontal plane (these two planes define a patient specific coordinate system and thus the location of the patient's brain). This also avoids misuse of the helmet by children for example. In addition, there is no longer a need to check the correct electrode positions, because, as soon as the patient has mounted the helmet on his head, the electrode positions relative to the brain will be known and well-defined.

The invention can therefore be highly beneficial for enabling accurate electrical stimulation and recording of the brain for patients at home, i.e., outside of clinical environments and without the need for assistance from clinical staff.

To allow rapid delivery of such custom-designed electrode helmets, the shell may be fabricated using an additive manufacturing technique. For example, the shell may have been formed by 3D-printing or, preferably, by a laser-sintering process, as two possible examples of additive manufacturing techniques.

Moreover, the shell may have been fabricated and/or the geometry of the shell may be based on 3D design data that have been derived from patient-specific anatomical 3D data measured from a patient's skull. Such anatomical data can be measured in particular using a 3D-scanner or a medical imaging technique such as a CT-(computed tomography) or MRI-(magnetic resonance imaging)scan. This approach is particularly useful when using an additive manufacturing technique, because the design data specifying the geometry of the shell can be rapidly adapted based on such patient-specific anatomical 3D data and applied to the manufacturing. Moreover, the process of adapting the geometry of the helmet to a patient's anatomy can be fully automated.

As a result, the shell, in particular an interior surface of the shell facing the patient's skull, can show a patient-specific shape. This enables the patient, for whom the helmet has been designed and fabricated, to wear the helmet with comfort and ease over hours. In addition, due to the patient-specific shape and the shape stability of the helmet, the helmet can be designed such that it can only be worn in a defined orientation with respect to the skull of the patient for whom the helmet is designed. In other words, when the particular patient wears such a helmet, the position of the electrodes carried by the helmet will be fixed w.r.t the skull, in particular w.r.t. to said sagittal and frontal plane.

Suitable materials for the shell are polymers, preferably of medical grade and/or suitable for prolonged skin contact, most preferably polyamide, but also soft, rubber like materials resulting in something like a 3D printed swimming cap with embedded electrodes; in the latter case, the required structural strength for providing the desired shape stability of the helmet may be provided by a skeleton that is stable in shape, and the flexible cap may be attached to that skeleton to form the shell.

According to another approach, which may be used to achieve the afore-mentioned objectives, the invention proposes a patient-specific flexible cap, which comprises a flexible shell layer (that is not stable in shape) designed for carrying a number of *m* electrodes for electrically contacting the scalp of a patient wearing the cap. This cap can thus offer a patient-specific design, in particular according to data which specify the anatomy of a skull of an individual patient for whom the cap is intended. As for the helmet, the design of such a cap can be based on 3D design data that have been derived from patient-specific anatomical 3D data measured from a patient's skull, in particular using a 3D-scanner or a medical imaging technique and/or the shell layer of the cap may be fabricated using an additive manufacturing technique. Of course, all other features of the helmet according to the invention, in particular with respect to electrode holders, electrode design, integrated wiring and other features of the helmet, can also be implemented in such a cap. For example, the cap may feature single areas which provide some structural strength (e.g., in the form of (curved) plate-like stiff elements integrated into the flexible cap) sufficient to carry an electrode holder that can produce a contact force for pressing an electrode of the cap into the scalp of the patient wearing the cap.

The shell of the mentioned helmet (or parts of the shell) can be formed with high precision and at high speed from the materials mentioned above using additive manufacturing techniques such as laser sintering, which is ideally suited for delivering a helmet with patient-specific design and dimensioning. Another advantage of this approach is that ventilation openings can be easily integrated in the shell of the helmet during the additive manufacturing. No extra processing is needed to define the openings. The helmet can thus feature ventilation openings which are defined by the shell.

According to one embodiment, which offers significant advantages in terms of fabrication time and cost, the shell may comprise (in particular consist) of at least two shell parts. Most preferably, each shell part may have been fabricated using an additive manufacturing technique, as described before. For example, in the simplest case, the shell may be composed of two shell halfs, for example each fabricated by laser sintering. As a result, during fabrication several shell halfs may be stacked on top of each other and fabricated in the same additive manufacturing run. As the shell halfs can be fabricated in an orientation different from an orientation which they have later when assembled to form the shell of the helmet, even the shell halfs of the same patient-specific helmet may be stacked (similar to a stack of soup plates) and fabricated in the same run using an additive manufacturing technique.

The shell of the helmet may thus be assembled from at least two shell part. These parts may be mechanically joined together to form the shell. Particularly when using a laser-sintering process, this has the advantage that the helmet parts can be stacked inside each other, allowing numerous helmet parts to be additively manufactured in parallel and simultaneously in a batch process. This approach can significantly speed up fabrication and lower fabrication costs.

When the shell is assembled from several parts, it may be preferable if a separation line separating the parts of the shell is not formed in a straight line. Rather, a S-shape of the separation line can be more suitable. In particular, the separation line may run in between electrodes carried by a left-hand shell part and corresponding electrodes carried by a right-hand part of the shell.

Of course, it is preferable when the shell parts are interconnected to form the shell in such a way that their relative position to each other is fixed. This way, each shell part can be used as a counterpart capable of providing accurate contact forces for pressing the electrodes into the scalp of the patient (which will be detailed below).

As already mentioned, the helmet may feature or be equipped (after fabrication) with a number of non-invasive electrodes designed for electrical stimulation and/or recording.

As already mentioned, the helmet features a number of N electrode holders and each holder is designed for holding a respective electrode in place. In detail, the holders can be designed and each oriented such that a respective electrode can be held by each holder and/or that a contact area of the electrode (design for direct skin contact/contact to the scalp) when held by the respective holder is facing radially inward with respect to a center of the helmet. In other words, each holder may be oriented to hold the respective electrode (when the patient is wearing the helmet) such that the electrode is oriented normally with respect to an inner surface of the helmet running tangential to the surface of the skull of the patient. Such a design enables a good contact of the electrodes to the scalp, in particular when using brush electrodes, as it will be safeguarded that the tips of all brushes of the respective electrode are in touch with the scalp.

The approach of equipping the helmet with dedicated electrode holders has the advantages that the electrodes do not need to be integrated permanently into the helmet but can be designed as replacement parts. When an electrode wears out or is defect, it can thus be simply replaced. When a sufficient number of the holders is equipped with a stimulation and/or recording electrode, the helmet can be employed as an electrical stimulation and/or recording device, when worn by a patient on his head. However, not all of the holders may need to be equipped with an electrode, depending on the needs of the particular patient.

Most preferably, the holders may be formed as integral parts of the shell. Hence, the holders may be fabricated simultaneously with the shell using an additive manufacturing method.

Depending on the design, the electrodes can alternatively be formed as integral parts of the helmet and permanently attached to the shell, in particular to said holders. This is particularly useful if the helmet features an integrated electrical wiring (which may be embedded into or deposited on the shell) for providing electrical contact to each electrode.

As already mentioned, each of said holders features a mechanical spring for providing a contact force. The spring, which may have the form of a coil spring or a bending beam or bending flap, can be designed such that the mentioned contact force is suitable for pressing an electrode held by the holder into the scalp of the patient wearing the helmet. Of course, this force will depend on the specific anatomy of the patient's skull, which can be taken into account during design and fabrication of the shell. Most preferably, the springs can also be formed as integral parts of the respective holder and/or the shell. Such a spring may thus be used for suspending a respective electrode to be carried by the respective holder / by the shell.

For example, one embodiment suggests designing each holder in the form of a flap that is one-sidedly connected to the shell and/or monolithically formed with the shell. Such a flap may have a triangular, rectangular or elliptical shape, for example. Such a bendable flap can be flexed and can thus provide the desired contact force for pushing the electrode. The orientation of the flaps may vary across the inner surface of the helmet.

Each holder, in particular said flap, can also feature an insertion opening for introducing an electrode (formed as a separate exchangeable part) and/or a mounting surface to be brought into contact with a counter surface of the electrode. Via the mounting surface, the flap/holder may thus transfer the contact force to the electrode, as soon as the flap/holder is deflected from its resting position (e.g., radially outwards w.r.t. to a center of the helmet).

As an alternative, conventional spring elements such as metallic or plastic coil or beam springs or the like may be used and arranged such that they can be loaded when displacing the respective holder.

In a rest position (when the spring is in equilibrium, i.e., without external forces applied), each holder may show a negative deflection Δr (i.e., a deflection in negative radial direction) from a contact position defined by a contour surface of the patient's skull for whom the helmet has been specifically designed. When the patient puts on the helmet, each holder may thus be deflected from the respective rest position outwards, against the force of the respective spring. As a result, an electrode mounted to one of the holders will be pressed onto the skull with a defined force as soon as the patient puts on the helmet. If no electrode is mounted on a particular holder, this holder may be inactive in the sense that it will not be deflected and thus exert no contact force, when the patient is wearing his/her helmet.

As a result, when the patient is wearing the helmet and her/his skull occupies said contour surface, the respective electrode holder equipped with an electrode will be deflected from it's rest position outwardly and the mechanical spring will provide a contact force directed towards the skull to the holder and thereby to the electrode. This contact force presses the respective electrode, which is held by the holder, into contact with the scalp of the patient. This approach can result in an excellent skin contact.

To improve the wearing comfort for the patient and to avoid excessive contact pressure and possibly pressure marks in the scalp, the contact force (and resulting contact pressure) delivered to each of the electrodes can be adjusted from helmet to helmet, taking into account the age of the patient and/or shape of her/his skull and or her/his hair volume. Accordingly, the design data on the basis of which the helmet has been fabricated by additive manufacturing can include design features or parameters that control the contact force provided by the respective spring. For example, variation of the contact force may be achieved by varying the stiffness (e.g., by variation of the thickness and/or width and/or number of or size of at least one indentation(s) used to lower the stiffness of the respective spring) and/or geometry (in particular size) of the mechanical spring and/or by changing said amount of deflection present in the rest position (e.g., by changing the rest position w.r.t. to the shell / the scalp of the patient). The latter approach is most easily accomplished using additive manufacturing techniques because the 3D design data can be easily adjusted to adjust the travel of the holder and/or spring and thereby the contact force produced as soon as the patient (whose skull anatomy is well known) puts on her/his helmet.

The individual contact force/contact pressure delivered by one of the springs can be precisely adjusted by the geometry of the springs and/or the holder. The contact pressure may thus be varied from patient to patient. Furthermore, a comparatively low contact pressure can be selected for the forehead, for example, while a higher contact force may be used at the back of the head.

According to an advanced embodiment, at least some of the electrode holders may be equipped with a vibrational actuator (which are used in smart phones) capable of transmitting a mechanical vibration to the electrode held by the respective holder. Such a design is beneficial because the electrodes, in particular the tips of a brush electrode, can better penetrate the hairs on the skull of the patient when being actively actuated. Such a vibrational actuation of the electrodes may also be used during stimulation therapy to lower the contact resistance, in particular when an electrical impedance of the electrode is measured to be too high for efficient therapy. Accordingly, the helmet may feature at least one vibrational actuator designed for actively vibrating one of the electrodes.

As a characteristic feature, the springs may be formed integrally with the shell, in particular from the same material and/or using the same additive manufacturing technique that is used for fabricating the shell. Accordingly, the springs may thus be connected to the shell via at least one flexure bearing (i.e., a "solid joint" or "monolithic hinge" with a degree of freedom allowing accurate movement / deflection of the spring without play).

The springs may be further characterized in that in their respective rest positions, the springs already show a deflection (e.g., a particular deflection angle). As a result, in the rest position, each electrode held by the respective holder may lie within an outer circumference defined by the patient's head for whom the helmet has been designed.

Following the above approaches, each spring may thus provide a patient- and/or location specific contact force that has been defined by the 3D design data employed in the additive manufacturing of the helmet / the shell.

For improving the ease of use of the helmet when using exchangeable electrodes with the helmet, one embodiment suggests that each holder features an exchangeable electrode connector. Preferably, each electrode connector may be attachable to the respective holder by a mechanical snap-in mechanism.

Each electrode connector can feature a socket designed for receiving and/or electrically contacting a contact pin of a respective electrode. The electrode connector can be electrically conductive at least in the area where the connector contacts the inserted electrode. The contact connector can thus be designed to electrically contact an electrode that is inserted into the socket.

Preferably the connector (and the holders) may be designed such that the connector can be inserted into the holder in an insertion direction and secured in place by inserting a contact pin of an electrode into the socket of the connector in a push direction running diagonally to the insertion direction.

Most preferably, the socket may feature a recess, preferably in the form of a groove, designed for interaction with a corresponding cross-sectional thickening of the contact pin of the electrode. Alternatively or additionally, the socket may feature a cross-sectional thickening designed for interaction with a corresponding recess of the contact pin of the electrode. In all of these cases, the electrode can be secured in place in a defined insertion depth inside the socket of the electrode connector.

According to another embodiment, the shell of the helmet may feature an integrated electrical wiring designed for electrically contacting electrodes to be carried by the shell (the electrodes may either be integral parts of the shell or designed as exchangeable electrodes).

For example, the helmet may feature an electrical cable connector that is electrically connected to the integrated wiring and which can be connected to a single but multi-core cable of an (external) electronic unit. This way, use of a large number of cables, as they are often used nowadays, can be avoided. Due to the integration of the wiring into the helmet, a much higher spatial density of electrodes can be implemented in the helmet, which is particularly interesting for high spatial resolution electrical recording, which may be beneficial for accurately monitoring brain waves of Epilepsy patients, for example.

According to an advanced embodiment, that requires no cables at all, the helmet may comprise a wireless communication interface (e.g., a bluetooth interface), for wireless communication with an external receiver unit, which may be part of an electronic unit, which will be described in more detail below. In addition, the helmet may comprise at least one (preferably digital) signal processor, configured to send out signals measured with the electrodes (in particular measured by one particular electrode) to the receiver unit and/or to receive commands via the communication interface and to deliver drive voltages corresponding to the command to the electrodes (in particular to one particular electrode). For example, according to one particular embodiment, each electrode may comprise a (preferably digital) signal processor capable of reading out electrical signals measured with that electrode (during recording of nerve signals from the brain) and/or capable of providing a drive voltage to that electrode (for electrostimulation). Each of these signal processors may use the wireless communication interface for communicating with the receiver unit. In other words, the receiver unit may read out and/or send commands to each signal processor wirelessly via the communication interface. In summary, each of the electrodes of the helmet may be controllable (electrical reading out and/or electrical driving) by a (in particular respective) signal processor built-into the helmet. Preferably, this processor may be capable of/configured to sending out measured data to and/or receiving control data from an external receiver unit via a wireless communication interface built-into the helmet.

The wiring can be embedded into the shell, which is advantageous, because in this case, the shell material can provide electrical insulation. Alternatively, the wiring may be deposited on a surface of the shell; in this case, insulation layers may be added on top of the wiring.

According to one particular embodiment, the wiring is first deposited on a flexible film and the film, together with the (thin film) wiring, is next deep drawn onto a surface of the shell, which may be preferably an inner surface of the shell (in particular located between an outer and inner hull of the shell).

One particular embodiment suggests that the wiring is fabricated, in particular together with at least a part of the shell, using an additive manufacturing technique. For example, the wiring may be directly 3d-printed or fabricated by laser sintering, each time using a powder that contains conductive particles.

One particular design suggests that the shell of the helmet comprises an inner hull and an outer hull, which are fabricated separately from each other. Using this approach, two different fabrication methods, in particular two differing additive manufacturing processes may be used for fabricating the inner and outer hull.

All of the above examples and features explained with respect to a wiring integrated into the helmet and intended to delivering electrical voltages to the electrodes may be used in combination. For example, it is possible to assemble the wiring on said flexible foil by additive manufacturing and to attach the foil to a hull forming a part of the shell.

Moreover, the mentioned inner hull may carry or form said holders and/or springs. The outer hull, by contrast, may carry said wiring. Of course, this approach may also be done vice versa, with the inner hull forming/carrying the holders and/or springs and the inner hull carrying the wiring.

In particular, said wiring may be fabricated by additive manufacturing together with the inner or outer hull (depending on the chosen design route). In both cases, it can be achieved that (in particular exchangeable) electrodes may be electrically contacted by the wiring and be held in place by the holders and/or be movably suspended by the springs.

As already mentioned, the helmet may feature a number of m non-invasive electrodes designed for electrical stimulation and/or recording. For example, in case the electrodes are designed as exchangeable parts, a number of m ≤ N of the holders may be equipped with a respective, individually addressable, non-invasive electrode. In other words, depending on the needs of the patient, not all of the holders necessarily need to be equipped with a respective electrode and hence there can be less electrodes than possible holders present.

Each of the electrodes (either implemented as integral parts of the helmet or as exchangeable parts) can show a contact area (to be brought into direct skin contact) with a diameter between 10-25 mm, preferably between 15-20 mm. Such values have been found to constitute an acceptable technical compromise when the electrodes are intended to be used both for stimulation and recording: On the one hand, it is advantageous to increase the diameter of the electrodes to reduce the electrical contact resistance (impedance). In electrostimulation applications, this impedance must be typically less than 10 kΩ / electrode. When recording nerve signals, on the other hand, an impedance of 150 kΩ may still be acceptable such that a smaller diameter may be used for the contact area, which will be beneficial for achieving a higher spatial density of the electrodes. Also, a larger diameter of the electrodes leads to a decrease in accuracy when controlling the direction of the currents employed for electrostimulation. However, it must be taken into account that the skull has numerous conductive and non-conductive layers, such that the electric fields generated by the electrodes can only be controlled with finite spatial accuracy, as the electric fields tend to "smear out" inside the brain.

To alleviate these challenges, one particular design proposes to employ brush electrodes. Brush electrodes are beneficial for performing electrostimulation on the head without the need of shaving off the hairs of the patient. Accordingly, the electrodes employed in the helmet may be designed as brush electrodes featuring flexible and conductive brush filaments for contacting the scalp of a patient. These brush filaments can penetrate even thicker layers of hair.

Preferably, each brush electrode may feature a feed channel for introducing a conductive gel. This feed channel may be designed such that it is easily accessible from the outside, when the helmet is worn by a patient. In such a case, the gel may be introduced from outside the helmet in between the brush filaments while a patient is wearing the helmet on her/his head. This way, a sufficiently low electrical contact resistance can be achieved, even when the electrode alone does not provide a good electrical contact to the scalp.

The electrodes may be designed, for example, such that each electrode features at least 36 brush filaments. This not only significantly increases the effective contact surface of the electrode in case of using such an electrode without a conductive gel (e.g., dry electrodes for EEG). When using such an electrode with a (electrolyte) gel (e.g., wet electrodes for electrostimulation), the contact area between electrode and gel will also be increased, which will also help in decreasing the interface impedance as well (although this secondary effect is less important than increasing the contact surface of the skin-electrode/gel interface).

The brush filaments may be arranged symmetrically with respect to a central axis of the electrode (which may correspond to an axis of the contact force applied to the electrode). Such a design is beneficial, because it will lead to a uniform distribution of an conductive gel introduced through a feed channel of the electrode. This can significantly decrease electrical noise during recording, and also helps to form uniform electric field distributions inside the brain during electrostimulation using the electrodes.

The brush filaments can also serve as a scaffold for holding a conductive gel in place. This prevents flowing / dropping of the gel especially in areas, in which dropping of the gel from the electrode would be otherwise driven by gravity (e.g., in posterior, temporal, on front head). The use of a high number of brush filaments capable of retaining the gel is also beneficial for preventing electrical short circuits between two neighboring electrodes, which is a common problem when using saline solutions. As a result, a high spatial resolution of recording and/or electrostimulation can be maintained when using a gel.

It is also suggested to fabricate the electrodes of the helmet by injection molding. In particular suitable electrodes may be formed from a flexible and conductive material, for example a rubber material mixed with conductive particles.

The electrodes, in particular if designed as brush electrodes, can feature microneedles (preferably arranged at the tips of the brush filaments), designed to penetrate the scalp, preferably up to a depth of not more than 300 um, preferably not more than 100 um. Such microneedles or microtips can be easily formed using suitable microstructures (as part of a mold tool), when fabricating the electrodes by injection molding. When the electrode contacts the scalp, the microneedles/ microtips will penetrate the superficial layers of the sculp which can improve the electrical contact resistance significantly.

Another approach proposed for lowering the impedance is to use an additional coating for reducing the electrical contact resistance to the skull. Accordingly, the electrodes may feature an outer conductive coating, e.g. a thin (< 100 nm thickness) metallic coating. To improve the robustness and use-time of such an improved electrode, it is suggested to deposit the coating on a micro-corrugation (preferably with dimensions below 100 µm), which is formed in a surface of a body of the electrode. This body may form said brush filaments and it is particularly useful to deposit such a coating onto the filaments. Such a coating can improve electrochemical conductivity and ion transition (ions in body/neural tissues are responsible for charge transfer).

According to a preferred design, the electrodes or at least said holders (intended for carrying a respective electrode) may be distributed on an interior side of the helmet above and below a transversal plane running at a height that corresponds to a height of the eyebrows of the patient (for whom the helmet has been designed), when the patient is wearing the helmet.

A helmet according to the invention may feature at least 2/5/10/20, or even at least 50 electrodes and/or electrode holders. If an integrated wiring is employed in the helmet, the number of electrodes that can be or are carried by the helmet may exceed 500 and may reach 1024.

According to a preferred embodiment, the helmet has retaining structures, which form an undercut below a transversal plane running through the center of the patient's ears, when the patient is wearing the helmet. Such retaining structures may be, in particular, designed in the form of cheek flaps and/or a neck support. The advantage of using such an undercut is that. Due to the flexibility of the shell of the helmet, the patient can bend the retaining structures outwards when putting on the helmet. Once the helmet is accurately in place, the retaining structures will impede relative movements between the helmet and the skull, in particular around the x/y/z-axes running through the center of the skull. Thereby, the xyy-position of the helmet will remain fixe relative to the skull and the positioning of the electrodes carried by the helmet relative to the skull will be safely maintained.

Following the concept presented at the beginning of providing custom-made patient specific electrode helmets, the invention also proposes a series of helmets, with each helmet of this series being designed and having features as defined in claim 1, which is directed towards a helmet. Moreover, each of the helmets of the series is based on a common design. This common design may define or comprise: (i) an identical number of electrode holders for each helmet; and/or (ii) the same type of exchangeable or integrated electrodes; preferably, each helmet of the series may have been fabricated using the same materials and/or same additive manufacturing technique or process.

However, to satisfy the needs of the individual patient, each helmet of the series differs from the other helmets of the series: (a) in a patient-specific geometry of the shell of the helmet and/or (b) in a patient-specific geometry of the springs; and/or (c) in a patient-specific contact force provided by the individual springs (i.e., a patient-specific distribution of contact forces within the helmet); and/or (d) in an arrangement of the electrodes; and/or(e) in an electrical wiring implemented in the helmet.

By such measures, personalized electrode helmets can be rapidly provided to patients according to medical needs and based on input from attending doctors and also based on anatomical 3D data measured from a patient's skull. The use of a common design, that is only adapted to an individual patient guarantees fast delivery, low costs, and high reliability and quality of the customized patient-specific helmets.

To ease the application of the invention to the benefit of the patient, the invention also proposes an electrical device, that can be used as an electrical stimulation device (for injecting electrical currents into the brain for stimulating brain tissue) and/or as an electrical recording device (for recording nerve signals from the brain). This device comprises a helmet according to claim 1; a number of m electrodes (these electrodes may be carried by the helmet or permanently integrated into the helmet); and an electronic unit that is connected to each of the *m* electrodes employed in the helmet.

The connection may be established by cables electrically or the connection may be established by a wireless control link, for example. In the first case (electrical connection), the electronic unit may be configured to provide electrical drive voltages to each of the electrodes and to detect electrical voltages recorded by the electrodes. In the second case (wireless connection), the electronic unit may be configured to read-out voltages recorded with/by the electrodes and/or to control electrical drive voltages applied to each of the electrodes. This control may be implemented using a wireless communication interface built-into the helmet. The drive voltages may be controlled and/or delivered by a processor (comprising electrical driving circuitry and an electrical power source) built-into the helmet.

The electronic unit can be integrated into the helmet. However, more suitable for easy production is a design in which the electronic unit is a separate component, most preferably designed as a wearable component. One particular embodiment suggests that the electronic unit is designed as a separate neck-band or neck clamp that can be worn on the neck by the patient who is wearing the helmet. The electronic unit may be connected by at least one cable to the electrode helmet. If the helmet features an integrated wiring connected to the m electrodes, the helmet may also feature a cable connector for connection with a cable of the electronic unit. This approach is advantageous because the same electronic unit may be used with different patient-specific helmets of the series previously explained.

According to one preferred embodiment, the electronic unit is configured to perform electrical impedance measurements using the electrodes carried by the helmet in reaction to a user input and to output a result of the impedance measurement to the user, in particular for each electrode used individually. Such an impedance measurement thus delivers information on how good the respective electrode is in electrical contact with the scalp of the patient. Based on the output from the electronic unit, which may be a sound / or visual output on a display, the user may thus be informed by the electronic unit which of the electrodes do not yet show a sufficiently low impedance (for example appropriate for efficient electrostimulation, i.e., for example below 15 kΩ). Accordingly, the user can then apply more conductive gel via the feed channel of the respective electrodes and/or readjust the electrode position slightly. The user can then trigger a second impedance measurement by another user input to the electronic unit (which may have a push-button or other input device designed for receiving said user input) to check whether the impedance is now low enough. This way, optimal conditions for successful stimulation and/or recording using the electrodes of the helmet can be achieved, in particular for each one of the electrodes. For optimum results, the frequency of the voltage used during the impedance measurement should match the frequency later used during stimulation and/or recording.

As has already been mentioned, the helmet can also comprise at least one (preferably a multitude of) vibrational actuator (which are often used in smart phones for providing haptical feedback) for actively vibrating one of the electrodes of the helmet. Such a vibrational actuator may be integrated in the shell of the helmet, in particular as part of the mentioned electrode holders. According to one embodiment, the electronic unit can therefore be configured to control and/or activate at least one vibrational actuator comprised in the helmet and designed to actively vibrate one of the electrodes. In particular, the electronic unit may activate a respective vibrational actuator in reaction to an electrical impedance that has been measured (e.g., by the electronic unit as described above) for the electrode (that can be actively vibrated by that particular actuator). Following this approach, the electronic unit may thus autonomously optimize the respective electrical contact between an electrode of the helmet and the scalp of the patient by actively vibrating the electrode, if the electrical impedance is determined by the electronic unit to be below a threshold.

In accordance with the present invention, there is also provided a method / a process, which solves the afore-mentioned problem. In particular there is provided a method for fabricating a shell of a helmet, which has features as defined in claim 1. The shell of the helmet is thus designed to carry a number of m electrodes which are intended for electrical stimulation and/or recording of the brain. The method comprises the following steps: fabricating the shell of the helmet in a patient-specific geometry, using an additive manufacturing technique, preferably 3D-printing or laser sintering.

As has been described above, this approach is particularly useful if the additive manufacturing is based on 3D design data that have been derived from patient-specific anatomical 3D data measured from a patient's skull. For example, the process of adapting a standard geometry of the helmet to the anatomy of the skull of an individual patient by adapting the relevant parts of a set of standard 3D design data of the helmet may be fully automated if a high-quality anatomical 3D data file describing the patient's skull is available.

Accordingly, the method can include a step of (i) measuring anatomical 3D data from a patient's skull, in particular using a 3D-scanner or a medical imaging technique. Furthermore, the method can include another step of (ii) computing a 3D design data file based on a standard design data file and taking into account the anatomical 3D data measured in step (i). Of course, the step of fabricating the shell in a patient-specific geometry can then be performed using said computed 3D design data file (which includes all relevant data required for fabricating the shell of the helmet). Most advantageously, the 3D design data file can be used as an input file for an additive manufacturing setup, such as a 3D-printer or laser sintering machine.

The 3D design data, which defines the patient-specific geometry of the helmet, may include at least one parameter that has been adapted based on patient-specific data, in particular said anatomical 3D data or for example data provided by a doctor treating or diagnosing the patient. The at least one parameter can comprise, for example:
(i) parameters which define an inner contour surface defined by an interior surface of the helmet; this contour surface can be tailor-made such that it closely matches the anatomy of the skull of the patient for whom the helmet is intended;
(ii) parameters which define at least one contact force, either provided by a respective mechanical spring fabricated as an integral part of the shell or delivered by a respective electrode holder (this holder may be loaded by a force that is provided by a separate spring element) fabricated as an integral part of the shell; each time, the contact force may be delivered to one of the electrodes of the helmet to bring this electrode in skin contact on the skull of the patient.
(iii) parameters which describe a respective location of the electrode holders and/or location of the electrodes;
(iv) parameters which describe the layout of an electrical wiring integrated into the shell and used for electrically connecting the electrodes; this latter approach is particularly useful if the common design used for fabricating the helmet does not vary the individual number and/or position of the electrode holders, because by custom-designing the wiring, the number and or correct position of electrodes electrically addressable via the wiring can be defined. It is also possible to provide markings on the helmet to indicate the positions, at which electrodes are to be assembled into the respective electrode holder for an individual patient, in the typical case where not all of the holders will be equipped with electrodes.

Preferred examples of the present invention will now be described in more detail, although the present invention is not limited to these examples. With reference to the accompanying drawings, where features with corresponding technical function are referenced with same numerals even when these features differ in shape or design:
- Fig. 1: shows an electrode helmet according to the invention in a perspective view,
- Fig. 2: shows a shell part of another helmet according to the invention in a partial cross-sectional view,
- Fig. 3: illustrates a side view on a brush electrode according to the invention,
- Fig. 4: presents a top view on the electrode of Figure 3,
- Fig. 5: presents a perspective view on the electrode of Figure 3,
- Fig. 6: is a partial cross-sectional view of the electrode of Figure 3,
- Fig. 7: is the same view as that of Figure 6, but now an conductive gel is introduced into a feed channel of the electrode,
- Fig. 8: illustrates details of an electrode holder used in the helmets presented in Figures 1 and 2,
- Fig. 9: shows a side view on another helmet according to the invention, which, together with a separate electronic unit, forms an electrical stimulation and recording device,
- Fig. 10: is a bottom view on another helmet according to the invention, and finally
- Fig. 11: presents a frontal view of the helmet of Figure 10.

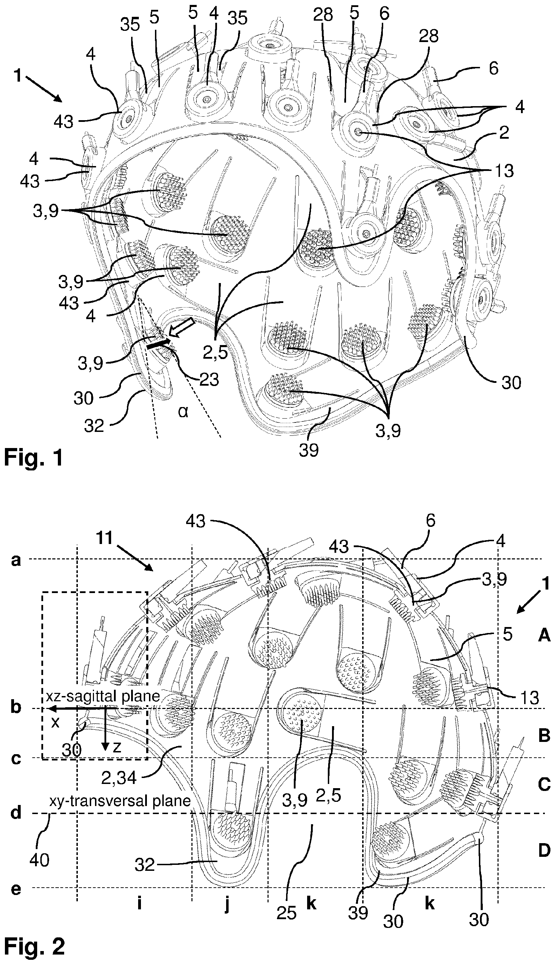

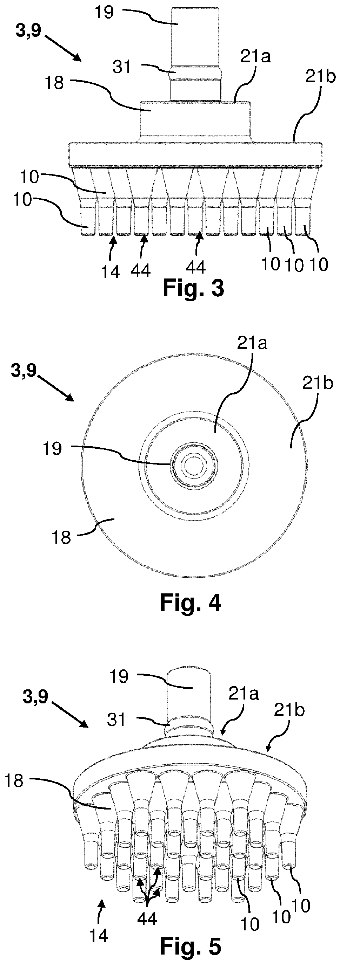

Figure 1 shows a first example of an electrode helmet 1 according to the invention, which has been fabricated according to a patient-specific design. The helmet 1 comprises a shell 2 which carries a number of electrodes 3. Each electrode 3 is designed for electrically contacting the scalp of a patient wearing the helmet 1 with a respective contact area 14 (cf. Figure 3). This helmet 1 can be used both for electrical stimulation of the brain and for recording of nerve signals from the brain, each time using the electrodes 3.

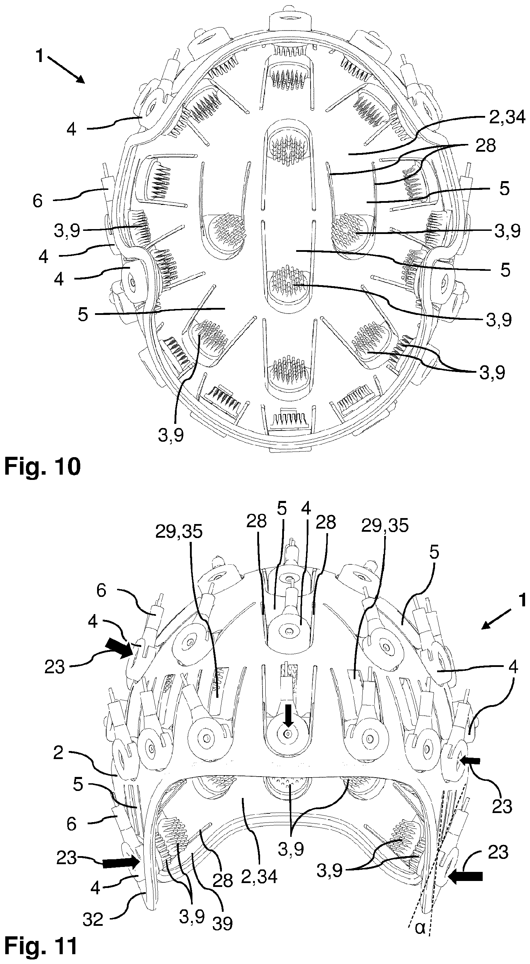

The shell 2 has been fabricated from a polyamide powder using a laser sintering machine, which makes it very easy to integrate ventilation openings 29 at desired locations (cf. Figure 11). The shape of the shell 2, in particular its inner contour surface 34, is based on a set of 3D design data. The 3D design data are based on a common design of the helmet 1, which includes details of the outer shape of the helmet and mechanical parts of the shell 2 such as the electrode holders 4. The holders 4 are formed and fabricated as part of the shell 2 and each designed for holding a respective one of the exchangeable electrodes 2.

Using a 3D-scanner, the head of the patient for whom the helmet 1 is intended was previously scanned to obtain patient-specific anatomical 3D data which characterize the shape of the skull of the patient, in particular its outer dimensions. Taking these anatomical 3D data into account, a 3D design data file was calculated and delivered to the laser sintering machines as an input file. Accordingly, the helmet 1 shows an inner contour surface 34 to be brought into contact with the scalp of the patient wearing the helmet 1 that is tailor-made to the patient's skull. The helmet 1 thus shows a patient-specific geometry that matches the geometry of the skull that was 3D-scanned. Thereby a high comfort of wearing is achieved.

The shell 2 is flexible but stable in shape, due to the solidity and flexibility of the polyamide. As a result, the position of each electrode 3 is well-defined with respect to a sagittal xy-plane and a frontal yz-plane running through the head of the patient wearing the helmet.

To avoid a dislocation of the helmet 1 relative to the skull during use, the helmet 1 features several retaining structures, namely two cheek flaps 32 and a neck support 39. These structures each form an undercut below a transversal xy-plane 40 (that is illustrated by the thick horizontal dashed line in Figure 2) which runs (horizontally) through the center of the patient's ears, when the patient is wearing the helmet 1. In the examples of Figures 1 and 2, the cheek flaps 32 and a neck support 39 even reach below the ears of the patient, which are positioned in the recesses 25 (cf. Figure 2) when the patient is wearing the helmet 1. Thanks to the undercut, any rotation or lateral movement of the helmet 1 relative to the skull is prevented. Moreover, due to the patient-specific design that follows the anatomy of the patient's skull, the helmet 1 can only be worn in one well-defined position and orientation. As a result, the positions of the electrodes 3 relative to the skull are fixed, as soon as the patient puts on the helmet 1. This is mainly achieved by the lower rim 30 of the helmet which follows the forehead, cheeks and neck of the patient (see quadrants Bi, Cj, Dj and Dk in Figure 2).

Figure 2 presents an example of a helmet 1 according to the invention in which the shell 2 is assembled from two shell parts 11, namely two shell halfs. Shown is only one of the two halfs 11. This approach can speed-up fabrication by additive manufacturing techniques such as laser sintering, because only one half of the shell 2 has to be produced at once. As several shell halfs 11 can be easily stacked, they can be fabricated in a batch process from the same polymer powder using one laser sintering run within a few hours.

As explained previously, the electrodes holders 4 of the helmet 1 are formed as integral parts of the shell 2 (cf. Figures 1 and 2). In addition, each holder 4 of the shell 2 features a mechanical spring 5 in the form of a flexible flap that can provide a contact force that will press the respective electrode 2 mounted in the holder 4 into the scalp on the skull of the patient wearing the helmet 1 (cf. Figures 1 and 2). As indicated by the dashed lines in Figure 1, in a rest position (i.e., without external forces applied) each of the holders 4 is slightly bend radially inwards. In other words, each holder 4 shows a negative deflection Δr (in radial direction) from a contact position defined by a contour surface of the patient's skull for whom the helmet 1 has been specifically designed. This deflection can also be described by the illustrated deflection angle α (cf. Figures 1, 8 and 11). Accordingly, the apices of the mounted electrodes 3 (in the rest position) would penetrate an imagined contour representing the outer surface of the skull of the patient - see Figure 1 or for example Figure 11.

When the patient puts on the helmet 1, each holder 4 is therefore deflected radially outward (as the skull pushes against the respective electrode 3) from the respective rest positions (i.e., the deflection angle α is lowered) - see for example Figures 1, 2, 8, 10 and 11 - against a force that is progressively produced by the respective spring 5, as the respective flap forming the spring 5 is bent outwards. As a result, an electrode 3 mounted to one of the holders 4 will be pressed onto the skull with a defined contact force 23 as soon as the patient puts on the helmet 1.

The amount of force that is produced can be fine-tuned by changing the amount of deflection (in particular said deflection angle α present in the rest position) and/or by changing the stiffness of the spring 5. Accordingly, the contact force 23 may vary from helmet 1 to helmet 1 but also with the position of the holder 4, as illustrated by the black arrows in Figure 11. For example, some of the springs 5 used may be weakened by thinning the shell 2 at their location or, as shown in Figure 11, by introducing recesses 5 that effectively reduce the cross-section and thereby the stiffness of the respective spring 5. The patient- and/or location specific contact force 23 that is provided by each spring 5 can be defined in the 3D design data employed in the additive manufacturing of the shell 2.

Figures 3 to 7 present further details of the electrodes 3 used together with the helmet 1. The electrodes 3 are designed as exchangeable brush electrodes 9 and have been fabricated by injection molding using a conductive polymer rubber mix. The molded body 18 of the electrodes 3 forms a number of flexible brush filaments 10. As the whole body 18 is electrically conductive, the electrodes 3 can be electrically contacted in the area of the illustrated contact pin 19.

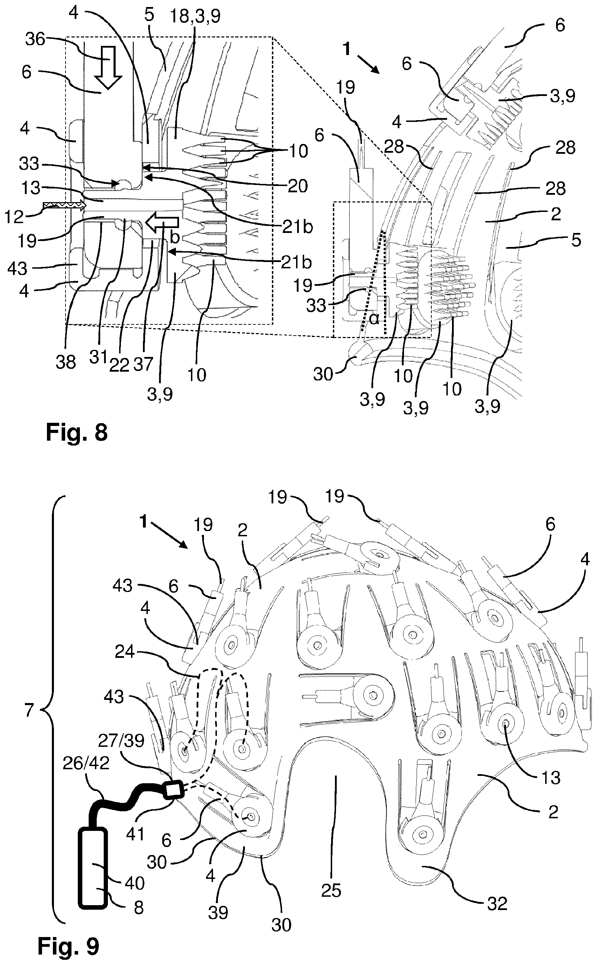

The contact pin 19 of the electrode body 18 features a cross-sectional thickening 31 (cf. Figures 3-7). As illustrated in Figure 8, this thickening 31 can interact with a corresponding groove 33 that is formed on an inner side of a socket 38 of a separate electrode connector 6.

The assembly of the electrodes 3 into the helmet 1 is performed as follows: First, the connectors 6 are slid into the respective holder 4 along the insertion direction 36 illustrated in Figure 8. Note that the holder 4 will transfer the contact force 23 provided by the spring 5 to the connector 6. Next, the brush electrode 3 is inserted from inside the helmet 1 into the connector 6 by pushing the electrode 3 radially outwards along the illustrated push direction 37 into the socket 38 of the connector 6 - see Figure 8.

Thanks to the thickening 31 formed at the contact pin 19 of the electrode 3, the latter will snap into the socket 38 and be fixed in position. A similar mechanical snap-in mechanism secures the connector 6 to the holder 4. Also note that the connector 6 is additionally secured in place by the inserted electrode 3, since the push direction 36 runs diagonally to the insertion direction 36. Finally, a respective electrical cable (not shown in the Figures) may be fitted to the outer end of the connector 6, which also forms a contact pin 19 (cf. Figures 8 and 9).

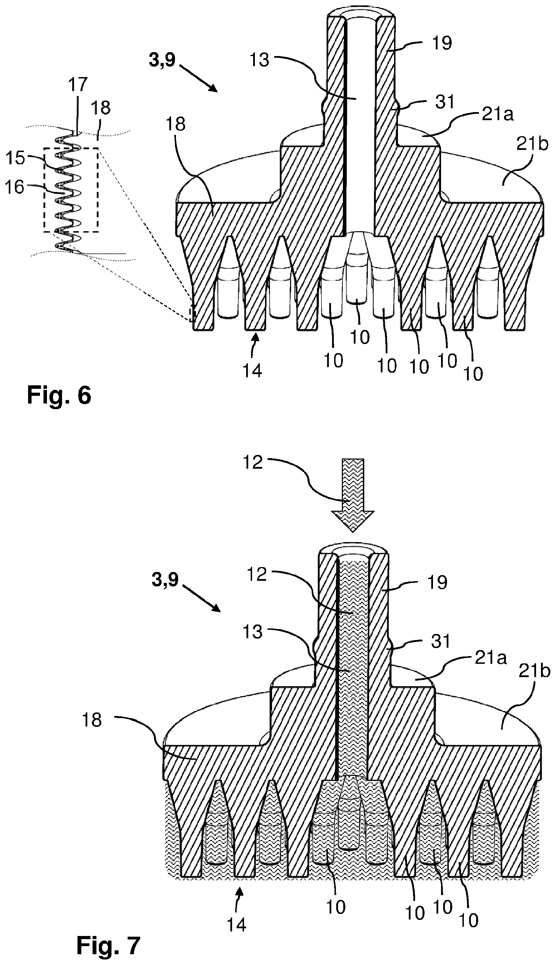

Figure 6 illustrates that the electrodes 3 may feature an outer conductive coating 15 for reducing the electrical contact resistance to the skull. This coating 15 has been deposited on a micro-corrugation 16 that is formed in a surface 17 of the body 18 of the electrode 2, in the region of the brush filaments 10.

Figure 7 illustrates the use of a conductive gel 12 that can be inserted into a feed channel 13 formed by the body 18 of the electrode 3. This way, the contact resistance of the electrode 3 in the contact area 14 can be lowered significantly. The symmetric positioning of the brush filaments 10 with respect to the feed channel 13 results in a uniform distribution of the gel 12 on the electrode 2 after injection of the gel 12 through the feed channel 13.

Figure 9 illustrates an alternative to connecting the connectors 6 (and thereby each electrode 3 contacted by the respective connector 6) to individual cables (resulting thus in a number of cables equal to the number m of electrodes 3 used in the helmet 1): The shell 2 of the helmet 1 can be equipped with an integrated wiring 4, as indicated by the dashed lines. This wiring 4, which may be embedded in the shell 2 and which may be formed by additive manufacturing techniques, can be designed to the needs of the patient such that all electrodes 3 present in the patient's helmet 1 are electrically contacted by the wiring 4. We note at this point, that depending on the region of the brain to be stimulated or recorded with the helmet 1, not all of the holders 4 necessarily need to be equipped with a corresponding electrode 3 (Hence some of the electrodes 3 shown in the Figures may be omitted, while it is easier for design and fabrication to maintain the holders 4).

In a more advanced version of the helmet 1, the cable 26 shown in Figure 7 may be replaced by a wireless (preferably bidirectional) wireless data connection 42. For this purpose, the electronic unit 8 may comprise a receiver unit 40 for communication with a wireless communication interface 39 built-into the helmet 1. A processor 41 (with additional electrical power source and driving circuitry) built-into the helmet 1 may then be used to read-out the electrodes 2 and/or to provide driving voltages to the electrodes 2, in particular according to commands received from the electronic unit via the wireless data connection 42.

In addition, the helmet 1 can feature a cable connector 27, as shown in Figure 9, to establish an electrical link between the integrated wiring 24 and an external electronic unit 8 via a (multi-core) cable 26. The electronic unit 8, which may be designed as a wearable neck-band, can be worn by the patient while the helmet 1 is mounted on his skull. The components shown in Figure 9 thus form an electrical device 7, which may be used as electrical stimulation and/or recording device 7 outside of hospitals by the patient himself.

As described in the claims and above, the electronic unit 8 may also be configured to perform impedance measurements using the electrodes 3 of the helmet to obtain a measure of the contact resistance of each of the electrodes 3. If the measured impedance is too high, more conductive gel 12 can be applied to the respective electrode 3 via the feed channel 13 from outside (i.e. the helmet 1 can remain in place on the skull).

In summary, an electrode helmet 1 and associated fabrication techniques have been proposed for simplifying the application of electrical stimulation and/or recording of the human brain for therapeutical or diagnostic purposes. The helmet 1 is stable in shape, can be designed to carry a varying number of m electrodes 3 and is characterized in that it features a patient-specific geometry that defines the relative position of each electrode with respect to the brain of the patient wearing the helmet 1. This approach improves the accuracy in stimulation and recording as well as the wearing comfort for the patient and allows tailor-made therapy and diagnostic with a helmet that can be customized at low costs based on a standard design and benefitting from accurate anatomical data obtained from a 3D scan or medical imaging of the patient's skull.

### List of reference numerals

- 1: helmet
- 2: shell
- 3: electrode
- 4: holder (for holding 3 in place)
- 5: spring / bending beam
- 6: electrode connector (for electrically contacting 3)
- 7: electrical stimulation and/or recording device
- 8: electronic unit
- 9: brush electrode
- 10: brush filament
- 11: part (forming 2)
- 12: conductive gel
- 13: feed channel
- 14: contact area (of 3)
- 15: conductive coating
- 16: micro-corrugation (formed in 17)
- 17: surface (of 18)
- 18: body (of 3, preferably injection-moulded)
- 19: contact pin (of 3 or 6)
- 20: mounting surface (formed on interior side of 4/2/32)
- 21: counter surface (of 3)
- 22: insertion opening (in 4 for introducing 3)
- 23: contact force
- 24: electrical wiring (for contacting 3)
- 25: recess (for the patient's ear)
- 26: cable (of 8 for electrical connection to 1/27/24)
- 27: cable connector (of 1, for connection to 26)
- 28: slit (in 2 for defining 5)
- 29: ventilation opening
- 30: (lower) rim (of 1)
- 31: cross-sectional thickening (of 18/19 of 3)
- 32: cheek flap (of 1/2)
- 33: groove (in 6 for interaction with 31)
- 34: inner contour surface (of 1 / 2)
- 35: recess / opening (for weakening 5)
- 36: insertion direction (when inserting 6 into 4)
- 37: push direction (when 3 / 19 is pushed into 6)
- 38: socket (of 6 for insertion of 3)
- 39: wireless communication interface (e.g., bluetooth)
- 40: receiver unit
- 41: processor
- 42: wireless data connection
- 43: vibrational actuator
- 44: microneedles

## Claims

1. **Patient-specific electrode helmet** (1) to be worn on the head, comprising
- a shell (2) designed for carrying a number of *m* electrodes (3) for electrically contacting the scalp of a patient wearing the helmet (1),
- wherein the shell (2) is stable in shape and offers a patient-specific design, **characterized in that**
- the helmet (1) features a number of N electrode holders (4), each holder (4) is designed for holding a respective electrode (3) in place, and
- each holder (4) features a mechanical spring (5) for providing a contact force (23) for pressing the electrodes (3) into the scalp of the patient.

2. Helmet (1) according to claim 1,
- wherein the shell (2) is designed according to data which specify the anatomy of a skull of an individual patient for whom the helmet (1) is intended,
- preferably such that the position of each electrode (3) is well-defined with respect to a sagittal and a frontal plane running through the head of the patient wearing the helmet (1) and/or
- wherein the holders (4) are formed as integral parts of the shell (2).

3. Helmet (1) according to claim 1 or 2,
- wherein the shell (2) has been fabricated using an additive manufacturing technique,
- preferably wherein the shell (2) has been formed by 3D-printing and/or by a laser-sintering process, and/or
- based on 3D design data that have been derived from patient-specific anatomical 3D data measured from a patient's skull, in particular using a 3D-scanner or a medical imaging technique.

4. Helmet (1) according to one of the preceding claims,
- wherein the shell (2) comprises at least two shell parts (11) that have each been fabricated using an additive manufacturing technique,
- preferably wherein the at least two shell parts (11) are interconnected to form the shell (2) such that their relative position to each other is fixed and/or
- wherein two of the at least two separate shell parts (11) are separated from each other by an S-shaped separation line,
- in particular wherein the separation line runs in between electrodes (3) carried by a left-hand shell part (11) and corresponding electrodes (3) carried by a right-hand shell part (11).

5. Helmet (1) according to the preceding claim,
- wherein the spring (5) of each holder (4) is designed such that the contact force (23) is suitable for pressing an electrode (3) held by the holder (4) into a scalp of a patient wearing the helmet (1) and/or
- wherein the springs (5) are formed as integral parts of the respective holder (4) and/or of the shell (2),
- most preferably wherein each spring (5) provides a patient- and/or location specific contact force that has been defined by 3D design data employed in the additive manufacturing of the shell (2).

6. Helmet (1) according to claim 1 or claim 5,
- wherein each holder (4) features an exchangeable electrode connector (6) with a socket (38) designed for receiving a contact pin (19) of a respective electrode (3),
- preferably wherein the connector (6) can be inserted into the holder (4) in an insertion direction (36) and secured in place by inserting a contact pin (19) of an electrode (3) into the socket (38) of the connector (6) in a push direction (37) running diagonally to the insertion direction (36),
- most preferably wherein the socket (38) features a recess, preferably in the form of a groove (33), or a cross-sectional thickening designed for interaction with a corresponding cross-sectional thickening (31) or recess of the contact pin (19) of the electrode.

7. Helmet (1) according to one of the preceding claims,
- wherein the helmet (1) features a built-in vibrational actuator (43) designed for actively vibrating one of the electrodes (2).

8. Helmet (1) according to one of the preceding claims,
- wherein the shell (2) features an integrated electrical wiring (24) designed for electrically contacting electrodes (2) to be carried or carried by the shell (2),
- in particular wherein the wiring (24)
- is embedded into the shell (2) or deposited on a surface of the shell (2) and/or
- has been fabricated using an additive manufacturing technique.

9. Helmet (1) according to one of the preceding claims,
- wherein each of the electrodes (2) of the helmet (1) is controllable by a signal processor (41) built-into the helmet (1),
- preferably wherein the processor (41) is configured to
- send out measured data to and/or
- receive control data from
an external receiver unit (40) via a wireless communication interface (39) built-into the helmet (1).

10. Helmet (1) according to the preceding claim,
- wherein the shell (2) features an inner hull and an outer hull fabricated separately, and
- wherein one of the two hulls forms or carries said holders (4) and/or said springs (5) and
- wherein the other hull carries said wiring (24).

11. Helmet (1) according to one of the preceding claims,
- wherein the helmet (1) features a number of *m* non-invasive electrodes (3) designed for electrical stimulation and/or recording, and/or
- wherein a number of *m* ≤ *N* of the holders (4) are equipped with a respective, individually addressable, non-invasive electrode (3).

12. Helmet (1) according to the preceding claim,
- wherein the electrodes (3) of the helmet (1)
- are designed as brush electrodes (9) featuring flexible and conductive brush filaments (10) for contacting the scalp of a patient and/or
- feature microneedles (44) designed to penetrate the scalp of the patient wearing the helmet (1) and/or
- wherein the electrodes (3) have been fabricated by injection molding, in particular from a flexible and conductive material, in particular a rubber material mixed with conductive particles,
- preferably wherein each brush electrode (9) features a feed channel (13) for introducing a conductive gel (12),
- in particular such that the gel (12) can be introduced from outside the helmet (1) in between the brush filaments (10) while a patient is wearing the helmet (1) on her/his head.

13. Helmet (1) according to claims 11 or 12,
- wherein the electrodes (2) feature an outer conductive coating (15) for reducing the electrical contact resistance to the skull,
- preferably wherein the coating (15) has been deposited on a micro-corrugation (16), preferably with dimensions below 100 um, formed in a surface (17) of a body (18) of the electrode (2).

14. Helmet (1) according to one of the preceding claims,
- wherein the helmet (1) has retaining structures (32, 39), in particular in the form of cheek flaps (32) and/or a neck support (39), which form an undercut below a transversal plane (40) which runs through the center of the patient's ears, when the patient is wearing the helmet (1) .

15. **Series of patient-specific electrode helmets (1),** wherein each helmet (1) of the series is
- designed according to one of the preceding claims and
- based on a common design,
- in particular with identical number of electrode holders (4) and/or
- comprises the same type of exchangeable or integrated electrodes (3), and/or
- with each helmet (1) having been fabricated using the same materials and/or same additive manufacturing technique,
- but differs from the other helmets (1) of the series in a patient-specific
- geometry of the shell (2) and/or
- geometry of the springs (5) and/or
- contact force (23) provided by the individual springs (5) and/or
- electrode (3) arrangements and/or
- electrical wiring (24) implemented in the helmet (1) .

16. **Electrical stimulation and/or recording device (7)** comprising
- a helmet (1) according to one of the claims 1 to 14,
- a number of *m* electrodes (2) carried by, in particular integrated into, the helmet (1), and
- an electronic unit (8) connected, either via a cable or wirelessly, to each of the *m* electrodes (3) and configured to
- provide or control electrical drive voltages to each of the electrodes (3) and/or
- to detect or read-out electrical voltages recorded by the electrodes (3),
- most preferably wherein the electronic unit (8) is designed as a separate neck-band or neck clamp to be worn on the neck by a patient wearing the helmet (1).

17. Device (7) according to the previous claim, wherein the electronic unit (8) is configured
- to perform electrical impedance measurements using the electrodes (3) in reaction to a user input and to output a result of the impedance measurement to the user and/or
- to control and activate at least one vibrational actuator (43) comprised in the helmet (1) and designed to actively vibrate one of the electrodes (2), in particular in reaction to a measured electrical impedance of that electrode (2).

18. **Method for fabricating a shell (2) of a helmet (1)** according to claim 1, the shell (2) being designed to carry a number of m electrodes (2) intended for electrical stimulation and or recording of the brain, **the method comprising the following steps,**
- fabricating the shell (2) in a patient-specific geometry, using an additive manufacturing technique, preferably 3D-printing and/or laser sintering;
- in particular based on 3D design data that have been derived from patient-specific anatomical 3D data measured from a patient's skull.

## Patentansprüche

1. **Patientenspezifischer Elektrodenhelm** (1), der auf dem Kopf zu tragen ist, umfassend
- eine Schale (2), die für das Tragen einer Anzahl von *m* Elektroden (3) zum elektrischen Kontaktieren der Kopfhaut eines/einer den Helm (1) tragenden Patienten/Patientin gestaltet ist,
- wobei die Schale (2) in ihrer Form stabil ist und eine patientenspezifische Gestaltung bietet, **dadurch gekennzeichnet, dass**
- der Helm (1) eine Anzahl von N Elektrodenhaltern (4) aufweist, jeder Halter (4) für das Halten einer jeweiligen Elektrode (3) an der Stelle gestaltet ist und
- jeder Halter (4) eine mechanische Feder (5) zum Bereitstellen einer Kontaktkraft (23) zum Drücken der Elektroden (3) in die Kopfhaut des Patienten/der Patientin aufweist.

2. Helm (1) nach Anspruch 1,
- wobei die Schale (2) in Abhängigkeit von Daten gestaltet ist, die die Anatomie des Schädels eines/einer individuellen Patienten/Patientin, für den/die der Helm (1) vorgesehen ist, vorgeben,
- sodass bevorzugt die Position jeder Elektrode (3) mit Bezug auf eine Sagittal- und eine Frontalebene, die durch den Kopf des/der den Helm (1) tragenden Patienten/Patientin verlaufen, eindeutig definiert ist, und/oder
- wobei die Halter (4) als integrale Bestandteile der Schale (2) gebildet sind.

3. Helm (1) nach Anspruch 1 oder 2,
- wobei die Schale (2) unter Nutzung einer additiven Fertigungstechnik hergestellt worden ist,
- wobei die Schale (2) bevorzugt durch 3D-Druck und/oder durch einen Lasersinterprozess gebildet worden ist und/oder
- basierend auf 3D-Gestaltungsdaten, die von anhand des Schädels eines Patienten/einer Patientin, insbesondere unter Nutzung eines 3D-Scanners oder einer medizinischen Bildgebungstechnik, gemessenen patientenspezifischen anatomischen 3D-Daten abgeleitet worden sind.

4. Helm (1) nach einem der vorhergehenden Ansprüche,
- wobei die Schale (2) mindestens zwei Schalenteile (11) umfasst, die je unter Nutzung einer additiven Fertigungstechnik hergestellt worden sind,
- wobei die mindestens zwei Schalenteile (11) bevorzugt miteinander verbunden sind, um die Schale (2) zu bilden, sodass ihre relative Position zueinander fest ist, und/oder
- wobei zwei der mindestens zwei separaten Schalenteile (11) durch eine S-förmige Trennungslinie voneinander getrennt sind,
- wobei die Trennungslinie insbesondere zwischen von einem linken Schalenteil (11) getragenen Elektroden (3) und entsprechenden von einem rechten Schalenteil (11) getragenen Elektroden (3) verläuft.

5. Helm (1) nach dem vorhergehenden Anspruch,
- wobei die Feder (5) jedes Halters (4) so gestaltet ist, dass die Kontaktkraft (23) zum Drücken einer von dem Halter (4) gehaltenen Elektrode (3) in die Kopfhaut eines/einer den Helm (1) tragenden Patienten/Patientin geeignet ist, und/oder
- wobei die Federn (5) als integrale Bestandteile des jeweiligen Halters (4) und/oder der Schale (2) gebildet sind,
- wobei jede Feder (5) am bevorzugtesten eine patienten- und/oder ortsspezifische Kontaktkraft bereitstellt, die durch bei der additiven Fertigung der Schale (2) verwendete 3D-Gestaltungsdaten definiert worden ist.

6. Helm (1) nach Anspruch 1 oder Anspruch 5,
- wobei jeder Halter (4) einen austauschbaren Elektrodenstecker (6) mit einer für das Aufnehmen eines Kontaktstifts (19) einer jeweiligen Elektrode (3) gestalteten Buchse (38) aufweist,
- wobei der Stecker (6) bevorzugt in einer Einführungsrichtung (36) in den Halter (4) eingeführt und an der Stelle befestigt werden kann, indem ein Kontaktstift (19) einer Elektrode (3) in die Buchse (38) des Steckers (6) in einer diagonal zu der Einführungsrichtung (36) verlaufenden Schubrichtung (37) eingeführt wird,
- wobei die Buchse (38) am bevorzugtesten eine Ausnehmung, bevorzugt in Form einer Rille (33), oder eine Querschnittsverdickung aufweist, die zur Wechselwirkung mit einer entsprechenden Querschnittsverdickung (31) oder Ausnehmung des Kontaktstifts (19) der Elektrode gestaltet ist.

7. Helm (1) nach einem der vorhergehenden Ansprüche,
- wobei der Helm (1) einen eingebauten Schwingungsaktor (43) aufweist, der so gestaltet ist, dass er eine der Elektroden (2) aktiv zum Schwingen bringt.

8. Helm (1) nach einem der vorhergehenden Ansprüche,
- wobei die Schale (2) eine integrierte elektrische Verdrahtung (24) aufweist, die für das elektrische Kontaktieren von von der Schale (2) getragenen oder zu tragenden Elektroden (2) gestaltet ist,
- wobei die Verdrahtung (24) insbesondere
- in die Schale (2) eingebettet oder auf einer Oberfläche der Schale (2) aufgebracht ist und/oder
- unter Nutzung einer additiven Fertigungstechnik hergestellt worden ist.

9. Helm (1) nach einem der vorhergehenden Ansprüche,
- wobei jede der Elektroden (2) des Helms (1) durch einen in den Helm (1) eingebauten Signalprozessor (41) steuerbar ist,
- wobei der Prozessor (41) bevorzugt für Folgendes konfiguriert ist:
- Aussenden von Messdaten an und/oder
- Empfangen von Steuerdaten von einer externen Empfängereinheit (40) über eine in den Helm (1) eingebaute drahtlose Kommunikationsschnittstelle (39).

10. Helm (1) nach dem vorhergehenden Anspruch,
- wobei die Schale (2) eine innere Hülle und eine äußere Hülle, die separat hergestellt werden, aufweist und
- wobei eine der zwei Hüllen die Halter (4) und/oder die Federn (5) bildet oder trägt und
- wobei die andere Hülle die Verdrahtung (24) trägt.

11. Helm (1) nach einem der vorhergehenden Ansprüche,
- wobei der Helm (1) eine Anzahl von *m* nicht invasiven Elektroden (3) aufweist, die zur elektrischen Stimulation und/oder Aufzeichnung gestaltet sind, und/oder
- wobei eine Anzahl *m* ≤ *N* der Halter (4) mit einer jeweiligen, individuell ansprechbaren, nicht invasiven Elektrode (3) ausgestattet ist.

12. Helm (1) nach dem vorhergehenden Anspruch,
- wobei die Elektroden (3) des Helms (1)
- als Bürstenelektroden (9) gestaltet sind, die flexible und leitfähige Bürstenfilamente (10) zum Kontaktieren der Kopfhaut eines Patienten/einer Patientin aufweisen, und/oder
- Mikronadeln (44) aufweisen, die zum Durchdringen der Kopfhaut des/der den Helm (1) tragenden Patienten/Patientin gestaltet sind, und/oder
- wobei die Elektroden (3) durch Spritzgießen, insbesondere aus einem flexiblen und leitfähigen Werkstoff, insbesondere einem mit leitfähigen Partikeln gemischten Gummiwerkstoff hergestellt worden sind,
- wobei jede Bürstenelektrode (9) bevorzugt einen Zuführungskanal (13) zum Einleiten eines leitfähigen Gels (12) aufweist,
- sodass das Gel (12) insbesondere von außerhalb des Helms (1) zwischen die Bürstenfilamente (10) eingeleitet werden kann, während ein Patient/eine Patientin den Helm (1) auf seinem/ihrem Kopf trägt.

13. Helm (1) nach Anspruch 11 oder 12,
- wobei die Elektroden (2) einen äußeren leitfähigen Überzug (15) zum Verringern des elektrischen Kontaktwiderstands gegen den Schädel aufweisen,
- wobei der Überzug (15) bevorzugt auf Mikroriffeln (16), bevorzugt mit Abmessungen von unter 100 µm, die in einer Oberfläche (17) eines Körpers (18) der Elektrode (2) gebildet sind, aufgebracht worden ist.

14. Helm (1) nach einem der vorhergehenden Ansprüche,
- wobei der Helm (1) Haltestrukturen (32, 39), insbesondere in Form von Wangenklappen (32) und/oder einer Halsstütze (39), aufweist, die einen Unterschnitt unter einer Transversalebene (40) bilden, die durch die Mitte der Ohren des Patienten/der Patientin verläuft, während der Patient/die Patientin den Helm (1) trägt.

15. **Reihe patientenspezifischer Elektrodenhelme (1),** wobei jeder Helm (1) der Reihe
- nach einem der vorhergehenden Ansprüche gestaltet ist und
- auf einer gemeinsamen Gestaltung basiert,
- insbesondere eine identische Anzahl von Elektrodenhaltern (4) aufweist und/oder
- denselben Typ austauschbarer oder integrierter Elektroden (3) umfasst, und/oder
- wobei jeder Helm (1) unter Nutzung derselben Werkstoffe und/oder derselben additiven Fertigungstechnik hergestellt worden ist,
- sich jedoch von den anderen Helmen (1) der Reihe unterscheidet, nämlich in einer patientenspezifischen
- Geometrie der Schale (2) und/oder
- Geometrie der Federn (5) und/oder
- Kontaktkraft (23), die von den individuellen Federn (5) bereitgestellt wird, und/oder
- Anordnungen von Elektroden (3) und/oder
- elektrischen Verdrahtung (24), die in dem Helm (1) implementiert ist.

16. **Vorrichtung (7) zur elektrischen Stimulation und/oder Aufzeichnung,** umfassend
- einen Helm (1) nach einem der Ansprüche 1 bis 14,
- eine Anzahl von *m* Elektroden (2), die von dem Helm (1) getragen werden, insbesondere in ihn integriert sind, und
- eine elektronische Einheit (8), die entweder über ein Kabel oder drahtlos mit jeder der *m* Elektroden (3) verbunden und konfiguriert ist, um
- elektrische Ansteuerspannungen für jede der Elektroden (3) bereitzustellen oder zu steuern und/oder
- durch die Elektroden (3) aufgezeichnete elektrische Spannungen zu erkennen oder auszulesen,
- wobei die elektronische Einheit (8) am bevorzugtesten als ein separates Halsband oder ein separater Halsbügel, das/der von einem/einer den Helm (1) tragenden Patienten/Patientin am Hals zu tragen ist, gestaltet ist.

17. Vorrichtung (7) nach dem vorherigen Anspruch, wobei die elektronische Einheit (8) für Folgendes konfiguriert ist:
- Durchführen von Messungen der elektrischen Impedanz unter Nutzung der Elektroden (3) als Reaktion auf eine Benutzereingabe und Ausgeben eines Ergebnisses der Impedanzmessung an den Benutzer/die Benutzerin und/oder
- Steuern und Einschalten mindestens eines Schwingungsaktors (43), der in dem Helm (1) umfasst und so gestaltet ist, dass er eine der Elektroden (2) aktiv zum Schwingen bringt, insbesondere als Reaktion auf eine gemessene elektrische Impedanz dieser Elektrode (2).

18. **Verfahren zum Herstellen einer Schale (2) eines Helms (1)** nach Anspruch 1, wobei die Schale (2) für das Tragen einer Anzahl von m Elektroden (2), die zur elektrischen Stimulation und/oder Aufzeichnung des Gehirns vorgesehen sind, gestaltet ist, **wobei das Verfahren die folgenden Schritte umfasst:**
- Herstellen der Schale (2) in einer patientenspezifischen Geometrie unter Nutzung einer additiven Fertigungstechnik, bevorzugt von 3D-Druck und/oder Lasersintern;
- insbesondere basierend auf 3D-Gestaltungsdaten, die von anhand des Schädels eines Patienten/einer Patientin gemessenen patientenspezifischen anatomischen 3D-Daten abgeleitet worden sind.

## Revendications

1. Casque à électrodes spécifique à un patient (1) à porter sur la tête, comprenant
- une coque (2) conçue pour porter un certain nombre m d'électrodes (3) destinées à venir en contact électriquement avec le cuir chevelu d'un patient portant le casque (1),
- dans lequel la coque (2) est de forme stable et offre une conception spécifique à un patient, **caractérisé en ce que**
- le casque (1) présente un certain nombre N de supports d'électrode (4), chaque support (4) étant conçu pour maintenir une électrode respective (3) en place, et
- chaque support (4) présente un ressort mécanique (5) pour fournir une force de contact (23) afin de presser les électrodes (3) contre le cuir chevelu du patient.

2. Casque (1) selon la revendication 1,
- dans lequel la coque (2) est conçue selon des données qui spécifient l'anatomie d'un crâne d'un patient individuel auquel le casque (1) est destiné,
- de préférence de telle sorte que la position de chaque électrode (3) soit bien définie par rapport à un plan sagittal et à un plan frontal s'étendant à travers la tête du patient portant le casque (1) et/ou
- dans lequel les supports (4) sont formés en tant que parties intégrantes de la coque (2).

3. Casque (1) selon la revendication 1 ou 2,
- dans lequel la coque (2) a été fabriquée à l'aide d'une technique de fabrication additive,
- de préférence dans lequel la coque (2) a été formée par impression 3D et/ou par un processus de frittage au laser, et/ou
- basé sur des données de conception 3D qui ont été dérivées de données 3D anatomiques spécifiques à un patient mesurées à partir du crâne d'un patient, en particulier à l'aide d'un scanner 3D ou d'une technique d'imagerie médicale.

4. Casque (1) selon l'une quelconque des revendications précédentes,
- dans lequel la coque (2) comprend au moins deux parties de coque (11) qui ont chacune été fabriquées à l'aide d'une technique de fabrication additive,
- de préférence dans lequel les au moins deux parties de coque (11) sont interconnectées pour former la coque (2) de sorte que leur position relative l'une par rapport à l'autre est fixe et/ou
- dans lequel deux des au moins deux parties de coque séparées (11) sont séparées l'une de l'autre par une ligne de séparation en forme de S,
- en particulier dans lequel la ligne de séparation passe entre des électrodes (3) portées par une partie de coque gauche (11) et des électrodes correspondantes (3) portées par une partie de coque droite (11).

5. Casque (1) selon l'une quelconque des revendications précédentes,
- dans lequel le ressort (5) de chaque support (4) est conçu de telle sorte que la force de contact (23) soit appropriée pour presser une électrode (3) maintenue par le support (4) contre le cuir chevelu d'un patient portant le casque (1) et/ou
- dans lequel les ressorts (5) sont formés en tant que parties intégrantes du support respectif (4) et/ou de la coque (2),
- de la manière la plus préférée dans lequel chaque ressort (5) fournit une force de contact spécifique à un patient et/ou à un emplacement qui a été définie par des données de conception 3D utilisées dans la fabrication additive de la coque (2).

6. Casque (1) selon la revendication 1 ou la revendication 5,
- dans lequel chaque support (4) présente un connecteur d'électrode interchangeable (6) avec une douille (38) conçue pour recevoir une broche de contact (19) d'une électrode respective (3),
- de préférence dans lequel le connecteur (6) peut être inséré dans le support (4) dans une direction d'insertion (36) et fixé en place en insérant une broche de contact (19) d'une électrode (3) dans la douille (38) du connecteur (6) dans une direction de poussée (37) s'étendant en diagonale par rapport à la direction d'insertion (36),
- de la manière la plus préférée dans lequel la douille (38) présente un évidement, de préférence sous la forme d'une rainure (33), ou un épaississement transversal conçu pour une interaction avec un épaississement transversal correspondant (31) ou un évidement de la broche de contact (19) de l'électrode.

7. Casque (1) selon l'une quelconque des revendications précédentes,
dans lequel le casque (1) comporte un actionneur vibrant intégré (43) conçu pour faire vibrer activement l'une des électrodes (2).

8. Casque (1) selon l'une quelconque des revendications précédentes,
- dans lequel la coque (2) comporte un câblage électrique intégré (24) conçu pour mettre en contact électriquement des électrodes (2) à porter ou portées par la coque (2),
- en particulier dans lequel le câblage (24)
- est implanté dans la coque (2) ou déposé sur une surface de la coque (2) et/ou
- a été fabriqué à l'aide d'une technique de fabrication additive.

9. Casque (1) selon l'une quelconque des revendications précédentes,
- dans lequel chacune des électrodes (2) du casque (1) peut être commandée par un processeur de signal (41) intégré dans le casque (1),
- de préférence dans lequel le processeur (41) est configuré pour
- envoyer les données mesurées à et/ou
- recevoir des données de commande à partir d'une unité de réception externe (40) via une interface de communication sans fil (39) intégrée dans le casque (1).

10. Casque (1) selon l'une quelconque des revendications précédentes,
- dans lequel la coque (2) comporte une coquille intérieure et une coquille extérieure fabriquées séparément, et
- dans lequel l'une des deux coquilles forme ou porte lesdits supports (4) et/ou lesdits ressorts (5) et
- dans lequel la coquille porte ledit câblage (24).

11. Casque (1) selon l'une quelconque des revendications précédentes,
- dans lequel le casque (1) comporte un certain nombre m d'électrodes non invasives (3) conçues pour une stimulation électrique et/ou un enregistrement, et/ou
- dans lequel un certain nombre de m ≤ N des supports (4) sont équipés d'une électrode non invasive adressable individuellement respective (3).

12. Casque (1) selon l'une quelconque des revendications précédentes,
- dans lequel les électrodes (3) du casque (1)
- sont conçues comme des électrodes à brosse (9) comportant des filaments de brosse souples et conducteurs (10) pour venir en contact avec le cuir chevelu d'un patient et/ou
- comportent des micro-aiguilles (44) conçues pour pénétrer dans le cuir chevelu du patient portant le casque (1) et/ou
- dans lequel les électrodes (3) ont été fabriquées par moulage par injection, en particulier à partir d'un matériau souple et conducteur, en particulier un matériau en caoutchouc mélangé à des particules conductrices,
- de préférence dans lequel chaque électrode à brosse (9) présente un canal d'alimentation (13) pour introduire un gel conducteur (12),
- en particulier de telle sorte que le gel (12) puisse être introduit depuis l'extérieur du casque (1) entre les filaments de brosse (10) pendant qu'un patient porte le casque (1) sur sa tête.

13. Casque (1) selon la revendication 11 ou 12,
- dans lequel les électrodes (2) comportent un revêtement conducteur extérieur (15) pour réduire la résistance de contact électrique avec le crâne,
- de préférence dans lequel le revêtement (15) a été déposé sur une micro-ondulation (16), de préférence avec des dimensions inférieures à 100 µm, formée dans une surface (17) d'un corps (18) de l'électrode (2).

14. Casque (1) selon l'une quelconque des revendications précédentes,
- dans lequel le casque (1) comporte des structures de retenue (32, 39), en particulier sous la forme de volets de joue (32) et/ou d'un support de cou (39), qui forment une contre-dépouille en dessous d'un plan transversal (40) qui s'étend à travers le centre des oreilles du patient, lorsque le patient porte le casque (1)

15. Série de casques à électrodes spécifiques à un patient (1), dans laquelle chaque casque (1) de la série est
- conçu selon l'une des revendications précédentes et
- basé sur une conception commune,
- en particulier avec un nombre identique de supports d'électrode (4) et/ou
- comprenant le même type d'électrodes interchangeables ou intégrées (3), et/ou
- avec chaque casque (1) ayant été fabriqué à l'aide des mêmes matériaux et/ou de la même technique de fabrication additive,
- mais diffère des autres casques (1) de la série, en termes de spécificité à un patient, par
- la géométrie de la coque (2) et/ou
- la géométrie des ressorts (5) et/ou
- la force de contact (23) fournie par les ressorts individuels (5) et/ou
- les agencements d'électrodes (3) et/ou
- le câblage électrique (24) mis en oeuvre dans le casque (1)

16. Dispositif de stimulation électrique et/ou d'enregistrement (7), comprenant
- un casque (1) selon l'une quelconque des revendications 1 à 14,
- un certain nombre m d'électrodes (2) portées par le casque (1), en particulier intégrées dans celui-ci, et
- une unité électronique (8) connectée, soit via un câble, soit sans fil, à chacune des m électrodes (3) et configurée pour
- fournir ou commander des tensions d'entraînement électrique à chacune des électrodes (3) et/ou
- détecter ou lire des tensions électriques enregistrées par les électrodes (3),
- de la manière la plus préférée dans lequel l'unité électronique (8) est conçue comme une bande de cou ou une pince de cou séparée à porter sur le cou par un patient portant le casque (1).

17. Dispositif (7) selon la revendication précédente, dans lequel l'unité électronique (8) est configurée
- pour effectuer des mesures d'impédance électrique à l'aide des électrodes (3) en réaction à une entrée d'utilisateur et pour fournir un résultat de la mesure d'impédance à l'utilisateur, et/ou
- pour commander et activer au moins un actionneur vibrant (43) compris dans le casque (1) et conçu pour faire vibrer activement l'une des électrodes (2), en particulier en réaction à une impédance électrique mesurée de cette électrode (2).

18. Procédé de fabrication d'une coque (2) d'un casque (1) selon la revendication 1, la coque (2) étant conçue pour porter un certain nombre m d'électrodes (2) destinées à une stimulation électrique et/ou à un enregistrement du cerveau, le procédé comprenant les étapes suivantes,
- la fabrication de la coque (2) dans une géométrie spécifique à un patient, à l'aide d'une technique de fabrication additive, de préférence une impression 3D et/ou un frittage laser ;
- en particulier sur la base de données de conception 3D qui ont été dérivées de données 3D anatomiques spécifiques à un patient mesurées à partir du crâne d'un patient.
